# EUROPÄISCHE PATENTSCHRIFT

(11) **EP 1 559 376 B1**
(45) Veröffentlichungstag und Bekanntmachung des Hinweises auf die Patenterteilung: **22.08.2007**
(21) Anmeldenummer: 04030803.3
(22) Anmeldetag: 27.12.2004
(51) Int. Cl.: A61B 17/56, A61B 17/88

(54) **Vorrichtung zum Einspannen von Knochenfixationsmitteln**
Device for clamping bone fixation means
Dispositif pour attacher des moyens de fixation osseux

(30) Priorität: 02.02.2004 DE 202004001504 U
(43) Veröffentlichungstag der Anmeldung: 03.08.2005
(73) Patentinhaber: Synthes GmbH, 4436 Oberdorf (CH)
(72) Erfinder: Schmucki, Daniel, 8143 Stallikon (CH); Frigeri, Patrizio, 8610 Uster (CH); Messmer, Peter, 8006 Zürich (CH)
(74) Vertreter: Lusuardi, Werther

(56) Entgegenhaltungen:
- US-A- 4 263 903
- US-B1- 6 673 078

## Beschreibung

Die Erfindung bezieht sich auf eine Vorrichtung zum Einspannen von Knochenfixationsmitteln, insbesondere Kirschnerdrähten gemäss dem Oberbegriff des Patentanspruchs 1.

Knochenfragmente, insbesondere am proximalen Femur sind oft schwierig so zu reponieren, dass eine interne Fixation der Fraktur möglich ist.

Eine Vorrichtung gemäss dem Oberbegriff von Anspruch 1 ist aus der US 6,673,078 MUNCIE und der US 4,263,903 GRIGGS offenbart.

Aus der WO 02/096294 GREEN ist eine Vorrichtung zur Ausrichtung von Röhrenknochen bekannt. Diese bekannte Vorrichtung umfasst einen Stab mit einem um einen Röhrenknochen herumführbaren, bogenförmigen Segment und einem Spannelement, welches parallel zum Stab verschiebbar angeordnet ist und gegen die proximale Oberfläche des Röhrenknochens pressbar ist. Nachteilig an dieser bekannten Vorrichtung ist, dass das bogenförmige Segment der Vorrichtung um den Röhrenknochen herumgeführt werden muss und dadurch eine Ablösung der umliegenden Weichteile notwendig ist.

Hier will die Erfindung Abhilfe schaffen. Der Erfindung liegt die Aufgabe zugrunde, eine Vorrichtung zu schaffen, welche am Körper des Patienten nur ein Einsetzen von wenigen Kirschnerdrähten erfordert, so dass nur kleine Inzisionen erforderlich sind und keine Ablösung der den Knochen umschliessenden Weichteile notwendig ist.

Die Erfindung löst die gestellte Aufgabe mit einer Vorrichtung zum Einspannen von Knochenfixationsmitteln, insbesondere Kirschnerdrähten, welche die Merkmale des Anspruchs 1 aufweist.

Die durch die Erfindung erreichten Vorteile sind im wesentlichen darin zu sehen, dass dank der erfindungsgemässen Vorrichtung
- sich der Klemmkörper beim gleichzeitigen Einspannen von Kirschnerdrähten mit unterschiedlichem Durchmesser zentrieren kann und die Kirschnerdrähte gleichmässig fest verspannt werden;
- nur kleine Inzisionen am Körper des Patienten erforderlich sind und keine Ablösung der den Knochen umgebenden Weichteile notwendig ist;
- die Kirschnerdrähte derart am Knochen befestigt werden können, dass sie nicht in den Markraum ragen oder diesen durchdringen, so dass ohne Entfernung der Vorrichtung ein Marknagel in den Markraum des zu behandelnden Knochens eingeführt werden kann; und
- die Kirschnerdrähte so am Knochen angeordnet werden können, dass ohne Entfernen der Vorrichtung eine Knochenplatte auf der Oberfläche des zu behandelnden Knochens plaziert werden kann.

In einer bevorzugten Ausführungsform umfassen die Spannmittel eine koaxial angeordnete Schraubverbindung zur axialen Verschiebung des Klemmkörpers relativ zum Hohlkörper. Dadurch ist der Vorteil erreichbar, dass ein grösserer axialer Verschiebebereich zwischen Klemmkörper und Hohlkörper möglich ist. Es können Kirschnerdrähte mit verschiedenen Durchmessern, beispielsweise innerhalb eines Durchmesserbereichs zwischen 1,0 mm und 3,5 mm eingesetzt werden.

In einer anderen Ausführungsform umfassen die Spannmittel zusätzlich oder anstelle der Schraubverbindung einen Exzentermechanismus, wodurch eine einfache Bedienung der Vorrichtung gewährleistet ist und hohe Klemmkräfte auf die einzuspannenden Kirschnerdrähte aufbringbar sind. Ferner dient der Exzentermechanismus als Sicherung, so dass kein unbeabsichtigtes Lösen der Spannmittel möglich ist. Falls die Spannmittel eine Schraubverbindung und einen Exzentermechanismus umfassen, ist der Exzentermechanismus unabhängig vom Durchmesser der einzuspannenden Kirschnerdrähte. Der Klemmkörper kann durch die Schraubverbindung so weit axial verschoben werden, bis seine Aussenwand die Kirschnerdrähte berührt und diese an der Hohlraumwand zur Anlage bringt.

In wiederum einer anderen Ausführungsform ist der Klemmkörper mindestens teilweise als konvexer Körper ausgebildet. Durch diese Ausgestaltung sind punktförmige Kontaktzonen zwischen den Kirschnerdrähten und dem Klemmkörper herstellbar, wodurch grössere Klemmkräfte auf die Kirschnerdrähte aufgebracht werden können.

In einer weiteren Ausführungsform weist der Klemmkörper auf Meridianen verlaufende Rillen auf. Damit ist der Vorteil erreichbar, dass die Kirschnerdrähte in den Rillen teilweise aufgenommen und seitlich geführt werden, so dass grössere Drehmomente um die Längsachse übertragbar sind.

In wiederum einer weiteren Ausführungsform weist der Klemmkörper einen gegen das hintere Ende des Hohlkörpers gerichteten, hinteren Abschnitt und axial entgegengesetzt einen vorderen Abschnitt auf, wobei sich der vordere Abschnitt und der hintere Abschnitt entgegengesetzt verjüngen. Vorzugsweise sind der hintere Abschnitt sphärisch konvex und der vordere Abschnitt des Klemmkörpers konisch ausgebildet.

Der Hohlkörper sowie der Klemmkörper weisen bevorzugt die folgenden Abmessungen auf:
- Konuswinkel α der konischen Hohlraumwand zwischen 5° und 35°;
- Abstand a zwischen 1,0 und 3,0 mm;
- Abstand A zwischen 3,0 und 6,0 mm; wobei
- das Verhältnis a : A der Abstände zwischen dem Klemmkörper und der konischen Hohlraumwand vorzugsweise zwischen 0,1 und 0,9 beträgt.

Weitere vorteilhafte Ausgestaltungen der Erfindung sind in den abhängigen Ansprüchen gekennzeichnet.

Die Erfindung und Weiterbildungen der Erfindung werden im folgenden anhand der teilweise schematischen Darstellungen mehrerer Ausführungsbeispiele noch näher erläutert.

### Es zeigen:

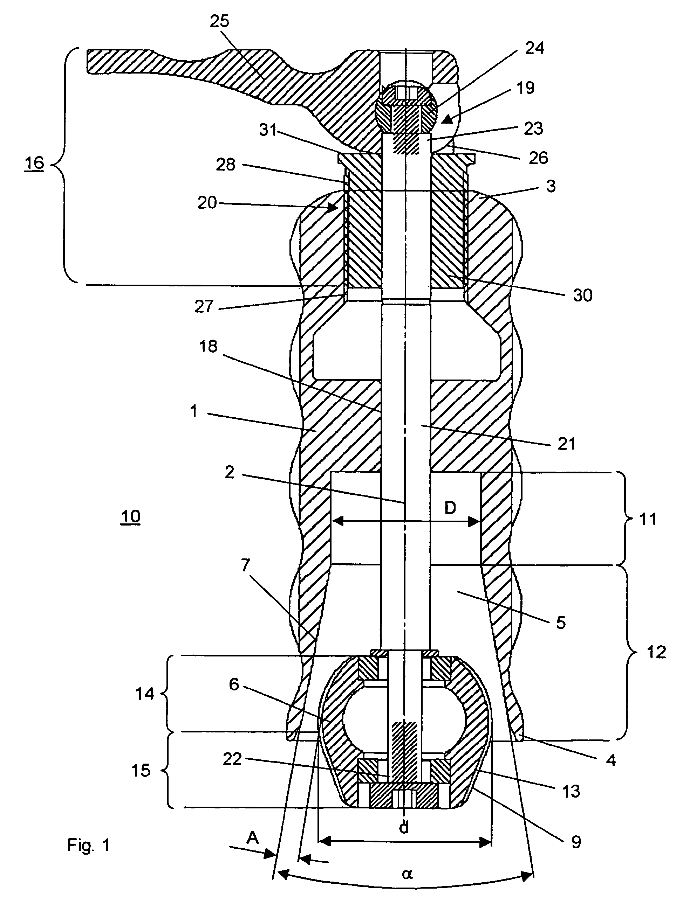

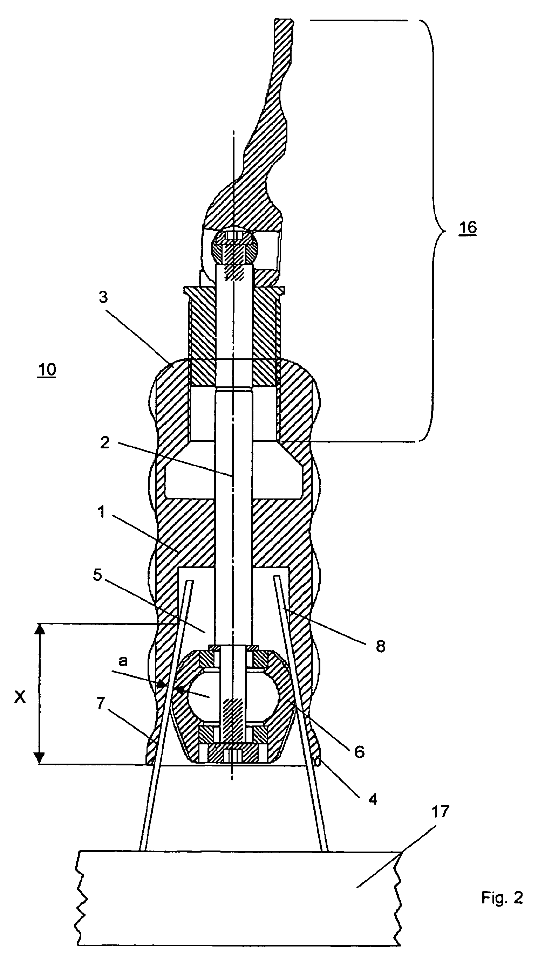

In den Fig. 1 und 2 ist eine Ausführungsform der Vorrichtung 10 dargestellt, welche im wesentlichen einen eine Längsachse 2 aufweisenden Hohlkörper 1, einen im Hohlraum 5 des Hohlkörpers 1 axial verschiebbaren und arretierbaren Klemmkörper 6 und am ersten Ende 3 des Hohlkörpers 1 angeordnete Spannmittel 16 umfasst. Im nicht blockierten Zustand der Vorrichtung 10 (Fig. 1) ist der Zwischenraum zwischen dem Klemmkörper 6 und der Hohlraumwand 7 soweit vergrössert, dass vom vorderen Ende 4 des Hohlkörpers 1 Kirschnerdrähte 8 am Klemmkörper 6 vorbei in den Hohlraum 5 eingeführt werden können. Der Hohlraum 5 weist ein koaxiales hohlzylindrisches Segment 11 und am vorderen Ende 4 des Hohlkörpers 1 ein ebenfalls koaxiales, konisches Segment 12 auf, wobei sich das konische Segment 12 gegen das vordere Ende 4 des Hohlkörpers 1 erweitert. Der minimale Innendurchmesser D des konischen Segmentes 12 entspricht dem Innendurchmesser des hohlzylindrischen Segmentes 11. Der Klemmkörper 6 ist konvex ausgebildet und weist einen maximalen Aussendurchmesser d auf, welcher grösser als der Innendurchmesser D des hohlzylindrischen Segmentes 11 ist. Durch axiales Verschieben des Klemmkörpers 6 gegen das hintere Ende 3 des Hohlkörpers 1 wird somit der kreisringförmige Spalt zwischen der Hohlraumwand 7 im konischen Segment 12 und der Aussenwand 9 des Klemmkörpers 6 verringert. Der Klemmkörper 6 wird beim Einspannen der Kirschnerdrähte 8 soweit gegen das hintere Ende 3 des Hohlkörpers 1 verschoben, bis die Kirschnerdrähte 8 im Spalt zwischen der Hohlraumwand 7 im konischen Segment 12 des Hohlraumes 5 und der Aussenwand 9 des Klemmkörpers 6 eingeklemmt werden (Fig. 2).

Die Aussenwand 9 des Klemmkörpers 6 weist gleichmässig auf dem Umfang verteilte, auf Meridianen verlaufende Rillen 13 auf, so dass die Kirschnerdrähte 8 parallel zur Längsachse 2 formschlüssig in diesen Rillen 13 gefangen sind. Zur einfacheren Einführung der Kirschnerdrähte 8 in den Spalt zwischen der Hohlraumwand 7 und der Aussenwand 9 des Klemmkörpers 6 ist der Klemmkörper 6 auf einem gegen das hintere Ende 3 des Hohlkörpers 1 gerichteten axialen Abschnitt 14 sphärisch und auf einem gegen das vordere Ende 4 des Hohlkörpers 1 gerichteten axialen Abschnitt 15 konisch ausgebildet.

Der Klemmkörper 6 wird mittels der Spannmittel 16 relativ zum Hohlkörper 1 verschoben und beim Einspannen der Kirschnerdrähte 8 axial fixiert. Dazu ist der Klemmkörper 6 am vorderen Ende 22 eines Stabes 21 gehalten, welcher in einer den Hohlkörper 1 koaxial durchdringenden Zentralbohrung 18 geführt wird. Der Klemmkörper 6 ist in einem bestimmten Mass quer zur Längsachse verschiebbar mit dem Stab 21 verbunden, so dass sich der Klemmkörper 6 beim gleichzeitigen Einspannen von Kirschnerdrähten 8 mit unterschiedlichem Durchmesser zentrieren kann. Der Stab 21 ist sowohl mittels des Exzentermechanismus 19 am hinteren Ende 23 des Stabes 21 als auch mit der Schraubverbindung 20 am hinteren Ende 3 des Hohlkörpers 1 axial bewegbar. Der Exzentermechanismus umfasst einen um eine zur Längsachse 2 orthogonale Drehachse 24 schwenkbar am Stab 21 befestigten Spannhebel 25 mit einer zur Drehachse 24 exzentrischen zylindrischen Kontaktfläche 26. Die axial verstellbare Schraubverbindung 20 ist so ausgebildet, dass die Zentralbohrung 18 am hinteren Ende 3 des Hohlkörpers 1 erweitert ist und ein Innengewinde 27 aufweist, in welches eine ein komplementäres Aussengewinde 28 aufweisende Hülse 30 geschraubt ist. Der Stab 21 wird axial durch die Hülse 30 durchgeführt und ist in seiner Länge derart bemessen, dass die Kontaktfläche 26 des Spannhebels 25 auf der axial aussenstehenden Stirnfläche 31 der Hülse 30 aufliegt.

Zur Anwendung der Vorrichtung 10 werden vom Chirurgen mindestens zwei, optimalerweise drei oder vier Kirschnerdrähte 8 in den Knochen 17 eingebohrt. Anschliessend wird der Exzentermechanismus 19 gelöst und die Hülse 30 in das Innengewinde 27 eingeschraubt bis der Klemmkörper 6 in einer vorderen Position ist, welche ein einfaches Einführen der hinteren Segmente der Kirschnerdrähte 8 in den Spalt zwischen Klemmkörper 6 und Hohlraumwand 7 erlaubt. Nach dem Einführen der hinteren Segmente der Kirschnerdrähte 8 in den Hohlraum 5 wird die Hülse 30 zurückgedreht bis die Kirschnerdrähte 8 leicht zwischen dem Klemmkörper 6 und der Hohlraumwand 7 verkeilt sind. Beim Spannen des Spannhebels 25 wird der Klemmkörper 6 durch den Exzentermechanismus 19 gegen die Kirschnerdrähte 8 gepresst, so dass diese fest im Spalt zwischen Klemmkörper 6 und Hohlraumwand 7 fixiert werden.

## Patentansprüche

1. Vorrichtung (10) zum Einspannen von an einem Knochen (17) befestigbaren Knochenfixationsmitteln, insbesondere Kirschnerdrähten (8), aufweisend
A) einen longitudinalen Hohlkörper (1) mit einer Längsachse (2), einem hinteren und einem vorderen die Längsachse (2) schneidenden Ende (3;4) und einem koaxialen, am vorderen Ende (4) des Hohlkörpers (1) offenen, sich gegen das hintere Ende (3) des Hohlkörpers (1) verjüngenden Hohlraum (5) mit einer mindestens auf einem Längenabschnitt X des Hohlraumes (5) konischen Hohlraumwand (7);
B) einen koaxial verschiebbaren, in den Hohlraum (5) einführbaren Klemmkörper (6), welcher sich gegen das hintere Ende (3) des Hohlkörpers (1) verjüngt; und
C) mit dem Klemmkörper (6) und dem hinteren Ende (3) des Hohlkörpers (1) verbundene Spannmittel (16), mittels welcher der Klemmkörper (6) relativ zum Hohlkörper (1) koaxial verschiebbar und in eine vordere Position und in eine hintere Position bringbar ist, wobei
D) die Spannmittel (16) mit dem Klemmkörper (6) mittels eines Stabes (21) verbunden sind, wobei
E) in der vorderen Position zwischen dem Klemmkörper (6) und der konischen Hohlraumwand (7) ein Abstand A besteht, welcher zum Einführen von Kirschnerdrähten (8) in den Hohlraum (5) geeignet ist; und
F) in der hinteren Position zwischen dem Klemmkörper (6) und der konischen Hohlraumwand (7) ein Abstand a < A besteht, welcher zum Verklemmen von Kirschnerdrähten (8) zwischen dem Klemmkörper (6) und der konischen Hohlraumwand (7) geeignet ist,
**dadurch gekennzeichnet, dass**
G) der Klemmkörper (6) quer zur Längsachse (2) verschiebbar mit dem Stab (21) verbunden ist.

2. Vorrichtung (10) nach Anspruch 1, **dadurch gekennzeichnet, dass** die Spannmittel (16) eine koaxial angeordnete Schraubverbindung (20) zur axialen Verschiebung des Klemmkörpers (6) umfassen.

3. Vorrichtung (10) nach Anspruch 1 oder 2, **dadurch gekennzeichnet, dass** die Spannmittel (16) einen Exzentermechanismus (19) umfassen.

4. Vorrichtung (10) nach einem der Ansprüche 1 bis 3, **dadurch gekennzeichnet, dass** der Klemmkörper (6) mindestens teilweise als konvexer Körper ausgebildet ist.

5. Vorrichtung (10) nach Anspruch 4, **dadurch gekennzeichnet, dass** der Klemmkörper (6) auf Meridianen verlaufende Rillen (13) aufweist.

6. Vorrichtung (10) nach einem der Ansprüche 1 bis 5, **dadurch gekennzeichnet, dass** der Klemmkörper (6) einen gegen das hintere Ende (3) des Hohlkörpers (1) gerichteten, hinteren Abschnitt (14) und axial entgegengesetzt einen vorderen Abschnitt (15) aufweist, wobei sich der vordere Abschnitt (15) und der hintere Abschnitt (14) entgegengesetzt verjüngen.

7. Vorrichtung (10) nach Anspruch 6, **dadurch gekennzeichnet, dass** der hintere Abschnitt (14) des Klemmkörpers (6) sphärisch konvex ausgebildet ist.

8. Vorrichtung (10) nach Anspruch 6 oder 7, **dadurch gekennzeichnet, dass** der vordere Abschnitt (15) des Klemmkörpers (6) konisch ausgebildet ist.

9. Vorrichtung (10) nach einem der Ansprüche 1 bis 8, **dadurch gekennzeichnet, dass** der Konuswinkel α der konischen Hohlraumwand (7) zwischen 5° und 35° beträgt.

10. Vorrichtung (10) nach einem der Ansprüche 1 bis 9, **dadurch gekennzeichnet, dass** das Verhältnis a : A der Abstände zwischen dem Klemmkörper (6) und der konischen Hohlraumwand (7) zwischen 0,1 und 0,9 beträgt.

## Claims

1. Device (10) for clamping bone fixation means, especially Kirschner wires (8), which can be fastened to a bone (17) comprising
(A) a longitudinal hollow body (1) with a longitudinal axis (2), a rear end and a front end (3; 4) intersecting the longitudinal axis (2) and a coaxial cavity (5) which is open at the front end (4) of the hollow body (1) and tapers towards the rear end (3) of the hollow body (1), with a conical wall (7) of the cavity at least on a longitudinal section X of the cavity (5);
(B) a coaxially displaceable clamping body (6), which can be introduced into the cavity (5) and tapers towards the rear end (3) of the hollow body (1) and
(C) clamping means (16) which are connected with the clamping body (6) and the rear end (3) of the hollow body (1) and by means of which the clamping body (6) can be shifted coaxially relative to the hollow body (1) and brought into a front position and into a rear position, whereby
(D) the clamping means (16) are connected with the clamping body (6) by means of a rod (21), whereby
(E) there being a distance A, which is suitable for introducing Kirschner wires (8) into the cavity (5), in the front position between the clamping body (6) and the conical wall (7) of the cavity; and
(F) there being a distance a < A, which is suitable for wedging Kirschner wires (8) between the clamping body (6) and the conical wall (7) of the cavity, in the rear position between the clamping body (6) and the conical wall (7) of the cavity,
**characterized in that**
(G) the clamping body (6) is connected with the rod (21) in such manner that it can be shifted transversely to the longitudinal axis (2).

2. The device (10) of claim 1, **characterized in that** the clamping means (16) include a coaxially disposed screw connection (20) for axially displacing the clamping body (6).

3. The device (10) of claims 1 or 2, **characterized in that** the clamping means (16) include an eccentric mechanism (19).

4. The device (10) of one of the claims 1 to 3, **characterized in that** the clamping body (6) is constructed at least partly as a convex body.

5. The device (10) of claim 4, **characterized in that** the clamping body (6.) has grooves (13) extending on meridians.

6. The device (10) of one of the claims 1 to 5, **characterized in that** the clamping body (6) has a rear section (14), directed towards the rear end (3) of the hollow body (1) and, axially opposite, a front section (15), the front section (15) and the rear section (14) tapering in opposite directions.

7. The device (10) of claim 6, **characterized in that** the rear section (14) of the clamping body (6) is constructed spherically convex.

8. The device (10) of claims 6 or 7, **characterized in that** the front section (15) of the clamping body (6) is constructed conically.

9. The device (10) of one of the claims 1 to 8, **characterized in that** the conical angle α of the conical wall (7) of the cavity is between 5° and 35°.

10. The device (10) of one of the claims 1 to 9, **characterized in that** the ratio a : A of the distances between the clamping body (6) and the conical wall (7) of the cavity is between 0,1 and 0,9.

## Revendications

1. Dispositif (10) pour serrer des moyens de fixation osseuse pouvant être fixés sur un os (17), en particulier des broches de Kirschner (8), comprenant
A) un corps creux longitudinal (1) présentant un axe longitudinal (2), des extrémités arrière et avant (3 ; 4) coupant l'axe longitudinal (2) et un espace creux (5) coaxial, ouvert au niveau de l'extrémité avant (4) du corps creux (1), se rétrécissant vers l'extrémité arrière (3) du corps creux (1), comprenant une paroi d'espace creux (7) conique au moins sur un segment longitudinal X de l'espace creux (5) ;
B) un corps de blocage (6) pouvant être inséré dans l'espace creux (5) et pouvant être déplacé coaxialement, lequel se rétrécit vers l'extrémité arrière (3) du corps creux (1) ; et
C) des moyens de serrage (16) reliés au corps de blocage (6) et à l'extrémité arrière (3) du corps creux (1), au moyen desquels le corps de blocage (6) peut être déplacé coaxialement au corps creux (1) et peut être mis dans une position avant et dans une position arrière, dans lequel
D) les moyens de serrage (16) sont reliés au corps de blocage (6) au moyen d'une tige (21), dans lequel
E) dans la position avant, il existe une distance A entre le corps de blocage (6) et la paroi d'espace creux conique (7), laquelle est appropriée à l'insertion de broches de Kirschner (8) dans l'espace creux (5) ; et
F) dans la position arrière, il existe une distance a < A entre le corps de blocage (6) et la paroi d'espace creux conique (7), laquelle est appropriée au blocage de broches de Kirschner (8) entre le corps de blocage (6) et la paroi d'espace creux conique (7),
**caractérisé en ce que**
G) le corps de blocage (6) est relié à la tige (21) de manière à pouvoir être déplacé transversalement à l'axe longitudinal (2).

2. Dispositif (10) selon la revendication 1, **caractérisé en ce que** les moyens de serrage (16) comprennent un raccord fileté (20) disposé coaxialement pour le déplacement axial du corps de blocage (6).

3. Dispositif (10) selon la revendication 1 ou 2, **caractérisé en ce que** les moyens de serrage (16) comprennent un mécanisme à excentrique (19).

4. Dispositif (10) selon l'une quelconque des revendications 1 à 3, **caractérisé en ce que** le corps de blocage (6) est réalisé au moins en partie sous forme de corps convexe.

5. Dispositif (10) selon la revendication 4, **caractérisé en ce que** le corps de blocage (6) présente des rainures (13) s'étendant sur des méridiens.

6. Dispositif (10) selon l'une quelconque des revendications 1 à 5, **caractérisé en ce que** le corps de blocage (6) présente un segment arrière (14) tourné vers l'extrémité arrière (3) du corps creux (1) et un segment avant (15) axialement opposé, le segment avant (15) et le segment arrière (14) se rétrécissant de manière opposée.

7. Dispositif (10) selon la revendication 6, **caractérisé en ce que** le segment arrière (14) du corps de blocage (6) est réalisé sous forme sphérique convexe.

8. Dispositif (10) selon la revendication 6 ou 7, **caractérisé en ce que** le segment avant (15) du corps de blocage (6) est réalisé sous forme conique.

9. Dispositif (10) selon l'une quelconque des revendications 1 à 8, **caractérisé en ce que** l'angle de cône α de la paroi d'espace creux conique (7) est compris entre 5° et 35°.

10. Dispositif (10) selon l'une quelconque des revendications 1 à 9, **caractérisé en ce que** le rapport a:A des distances entre le corps de blocage (6) et la paroi d'espace creux conique (7) est compris entre 0,1 et 0,9.
